Europäisches Patentamt

European Patent Office

Office européen des brevets

(19)

(11) Publication number: 0 269 200
A1

(12) **EUROPEAN PATENT APPLICATION**

(21) Application number: 87306809.2

(51) Int. Cl.4: **A61B 5/04 , A61N 1/04**

(22) Date of filing: 31.07.87

(30) Priority: 01.08.86 US 892805

(43) Date of publication of application:
01.06.88 Bulletin 88/22

(84) Designated Contracting States:
DE FR GB IT SE

(71) Applicant: **MINNESOTA MINING AND MANUFACTURING COMPANY**
**P.O. Box 33427**
**Saint Paul, MN 55133(US)**

(72) Inventor: **Pratt, Ronnie Lee Minnesota Mining and**
**Manufacturing Co 2501 Hudson Road P.O. Box 33427**
**St. Paul Minnesota 55133-3427(US)**
Inventor: **Roberts, Charles William Minnesota Mining and**
**Manufacturing Co 2501 Hudson Road P.O. Box 33427**
**St. Paul Minnesota 55133-3427(US)**
Inventor: **King-Smith, Eric A. c/o Minnesota Mining and**
**Manufacturing Co 2501 Hudson Road P.O. Box 33427**
**St. Paul Minnesota 55133-3427(US)**

(74) Representative: **Baillie, Iain Cameron et al**
**c/o Ladas & Parry Isartorplatz 5**
**D-8000 München 2(DE)**

(54) Flat biomedical electrode.

(57) A biomedical electrode (10) adapted to be applied to a body. A protective web (12) is coated with an electrically conductive adhesive (14) covering at least a portion of the protective web (12). A lead wire (18) having one end with an exposed electrically conductive portion (20) is positioned adjacent the electrically conductive adhesive (14) opposite the protective web (12). A removable liner (16) covers the electrically conductive adhesive (14). In one embodiment, an electrically insulating layer (26) is positioned adjacent the conductive portion (20) of the lead wire (18) opposite the electrically conductive adhesive (14) covering the exposed conductive portion (20) of the lead wire (18) but not covering the entire surface area of the electrically conductive adhesive (14). In one embodiment, an electrically conductive layer (24) is positioned between the protective web (12) and the electrically conductive adhesive (14) to promote current distribution over the entire surface area of the biomedical electrode (10). In an embodiment, a pressure sensitive adhesive (28) is also applied to the bottom side of the protective layer (12) to promote the adhesion of the protective layer (12) to the rest of the biomedical electrode (10).

EP 0 269 200 A1

FIG.1

# FLAT BIOMEDICAL ELECTRODE

## Technical Field

The present invention relates generally to biomedical electrodes.

## Background Art

Biomedical electrodes are useful for both stimulation and body monitoring functions. Stimulation uses of biomedical electrodes include transcutaneous electronic nerve stimulation (TENS) for the treatment of pain and neuromuscular stimulation (NMS) as, for example, treatment for scoliosis. Body monitoring uses for biomedical electrodes include electrocardiogram (ECG) for monitoring heart activity.

Among biomedical electrodes in existence are those of Phipps et al, Cartmell and Larimore. Phipps et al in United States Patent No. 3,170,459 discloses a biomedical instrumentation electrode constructed from multiple plies of discs made from a relatively inflexible material, i.e. cork. The electrode utilizes a conductive gel to establish contact with the body. Cartmell in United States Patent No. 4,543,958 discloses a medical electrode assembly. The electrode has a flexible, dimensionally stable substrate which is striped with an electrically conductive paint. The electrode is then clamped into a bulky cable connector. Larimore in United States Patent No. 4,458,696 (assigned to Minnesota Mining and Manufacturing Company) discloses a TENS electrode with a raised structure to permit entry of and attachment to a tubular electrical conductor.

These electrodes suffer from several deficiencies including that all are "high profile" electrodes and that the electrodes do not "conform" well to the body.

## Disclosure of Invention

The present invention provides a biomedical electrode which is (l) flatter and more conformable, (2) has a very low profile, (3) may be trimmed to differing shapes, (4) has flexibility in lead wire insertion direction, and (5) allows the reuse of the lead wire. The electrode of the present invention is flatter and more conformable to body contours and body movement than prior electrodes. The electrode relies on an adhesive contact with a flat electrical conductor as opposed to rubber connector strips or snaps. The electrode has a very low profile which makes it suitable to be worn under tight clothing and to be comfortable when slept upon or when leaned against, as for example, when sitting in a chair. The electrode may be trimmed to virtually any size or shape to allow adaptability in placement and location. The electrode allows for flexibility in lead wire insertion and allows for the lead wire to be inserted from either end of the electrode or from either side. The reuseability of the lead wire leads to economy for use of the biomedical electrode.

The present invention provides a biomedical electrode in which an electrical lead wire is secured to a body by an electrically conductive adhesive which is placed over the lead wire which in turn is topped by a protective, electrically insulative web. In a preferred embodiment, an electrically conductive film is utilized in conjunction with the conductive adhesive to help distribute the current over the entire surface area of the biomedical electrode. The protective web is secured to the electrically conductive film with a pressure sensitive adhesive. In an alternative embodiment, an insulator is provided between the uninsulated portion of the electrical lead wire and the body so that all the current to or from the electrical lead wire must travel through the electrically conductive adhesive to be disbursed over the surface area of the biomedical electrode.

The present invention also provides a biomedical electrode which is adapted to be applied to a body. The biomedical electrode has a protective web having a top side adapted to be oriented away from the body and a bottom side adapted to be oriented toward the body. The biomedical electrode has an electrically conductive adhesive positioned adjacent the bottom side of the protective web covering at least a portion of the protective web. The biomedical electrode also has a lead wire having one end with an exposed electrically conductive portion positioned adjacent the electrically conductive adhesive opposite the protective web. The biomedical electrode further has a removeable liner positioned adjacent to and covering the electrically conductive adhesive. An electrode constructed in this manner may have the removable liner removed and the biomedical electrode applied to the body. In a preferred embodiment, an electrically conductive layer is positioned between the protective web and the electrically conductive adhesive. The protective web is secured to the electrically conductive layer with a pressure sensitive adhesive. Also in a preferred embodiment, the protective web is also electrically insulative.

The present invention also provides a biomedical electrode adapted to be applied to a body. The biomedical electrode has a protective web having a top side adapted to be oriented away from the body and a bottom side adapted to be oriented toward the body. An electrically conductive adhesive is positioned adjacent the bottom side of the protective web covering at least a portion of the protective web. A lead wire having one end with an exposed electrically conductive portion is positioned adjacent to the electrically conductive adhesive opposite the protective web. An electrically insulative layer, is positioned adjacent that end of the lead wire opposite the electrically conductive adhesive in covering the exposed electrically conductive portion of the lead wire. A removable liner is positioned adjacent the electrically insulative web being secured by covering said electrically conductive adhesive. In a preferred embodiment, a layer of pressure sensitive adhesive is positioned between the bottom side of the protective web and the electrically conductive adhesive in order to better secure the protective web and the electrically conductive adhesive together.

## Brief Description of Drawings

The foregoing advantages, construction and operation of the present invention will become more readily apparent from the following description and accompanying drawings in which:

Figure 1 is an isometric view of the biomedical electrode of the present invention with the electrical lead wire;

Figure 2 is an expanded side view of one embodiment of the biomedical electrode of the present invention;

Figure 3 is an expanded side view of the biomedical electrode of the present invention showing the addition of an electrically conductive film;

Figure 4 is an expanded side view of an embodiment of the biomedical electrode of the present invention with an electrically conductive film and with an insulator covering exposed electrically conductive portion of the lead wire;

Figure 5 is an expanded side view of an embodiment of the biomedical electrode of the present invention showing the addition of a pressure sensitive adhesive to help secure the protective web.

## Detailed Description

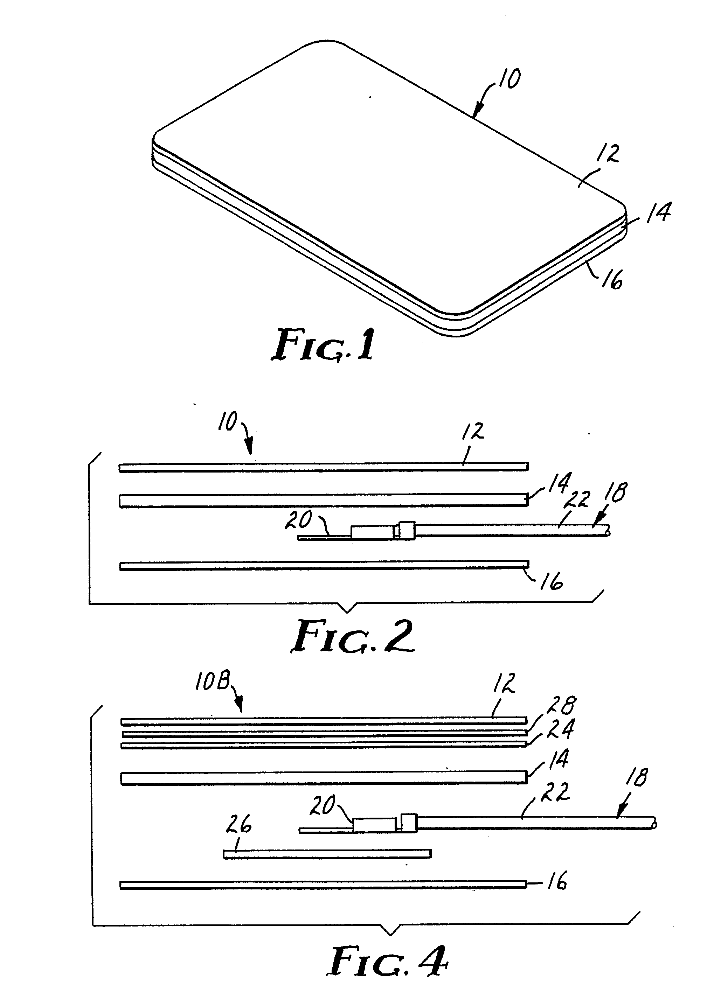

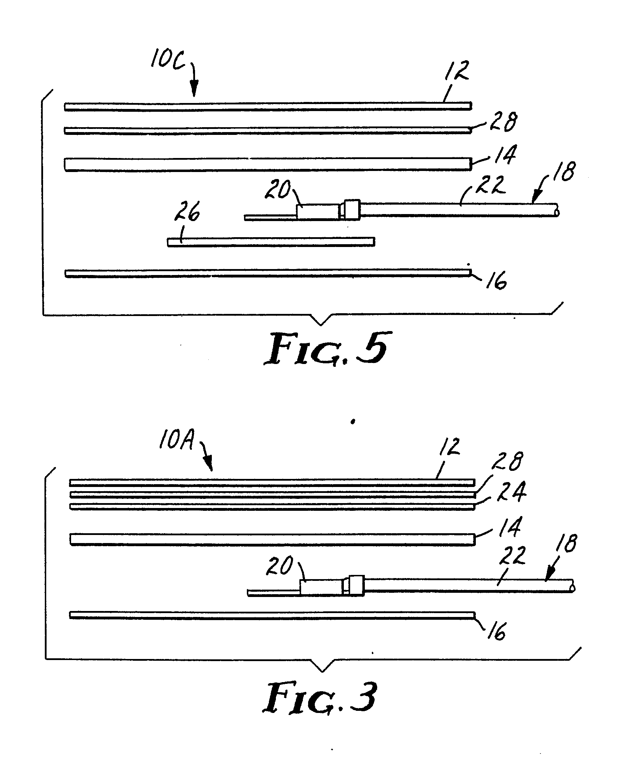

Figures 1 and 2 illustrate a preferred embodiment of the biomedical electrode 10 of the present invention. A protective web covering the top of the biomedical electrode 10 operates to protect the underlying layers of the biomedical electrode 10 from mechanical damage and also operates to carry conductive adhesive 14 before the biomedical electrode 10 is applied to the body (not shown).

Web 12 is electrically insulative to confine the electrical signals used in the biomedical electrode to the lead wire 18 or to the body (not shown). In a preferred embodiment, web 12 is approximately 4 mils (1.0 millimeter) thick and is constructed from a low molecular weight polyethylene film (reuseable electrode) or a spun bonded polyolefin (disposable electrode).

At least a portion of the surface area of protective web 12 is covered with an electrically conductive adhesive 14.

It is preferred that conductive adhesive 14 have better cohesion than adhesion in order to facilitate the ease in which the biomedical electrode 10 may be removed from the body. Conductive adhesive 14 may generally have a volume resistivity of the range from 50 to 200 ohms-centimeters although lower and higher volume resistivities may work for some uses of the biomedical electrode 10. In a preferred embodiment, conductive adhesive 14 is from 10 mils (2.5 millimeters) to 60 mils (15.2 millimeters) thick.

In a reuseable electrode it is preferred that the conductive adhesive 24 be from 30 mils (7.6 mm) to 44 mils (11.2 mm) thick. In a disposable electrode it is preferred that the conductive adhesive be from 10 mils (2.5 mm) to 15 mils (3.8 mm) thick. An example of a conductive adhesive 24 desireable for a disposable electrode is described in U.S. Patent No. 3,865,770, Blake, Water-Dispersable Pressure-Sensitive Adhesive, Tape Made Therewith, and Novel Tackifiers Therefor. It is preferred that the adhesive in Blake be modified and the following ingredients used:

3

| Ingredient | Dry Weight (Grams) | Dry Weight (Percent) |
|---|---|---|
| Copolymer: Butyl Acrylate & Acrylic Acid in 3:1 ratio | 83.177 | 40.945 |
| Glycerin | 20 | 9.8452 |
| 1,4 Butanediol | 20 | 9.8452 |
| Sorbitol | 20 | 9.8452 |
| Methyl diethanolamine (MDEA) | 28 | 13.783 |
| Potassium Chloride (KCl) | 6.9678 | 3.4300 |
| Foral AX (hydrogenated wood rosin) | 25 | 12.306 |
| Total | 203.14 | 100.04 |

An example of a conductive adhesive 24 desireable for a reusable electrode is described in U.S. Patent No. 4,554,924, Engel, Conductive Adhesive and Biomedical Electrode. It is preferred that the adhesive in Engel be modified and the following ingredients be used:

| Ingredient | Dry Weight (Grams) | Dry Weight (Percent) |
|---|---|---|
| 1,4 Butanediol | 45 | 6.3993 |
| Glycerin | 75 | 10.665 |
| Sorbitol | 290 | 41.240 |
| K739, Polyacrylic | 17 | 2.4175 |
| Potassium Hydroxide | 3.25 | 0.46217 |
| Total Water | 155 | 22.042 |
| Acrylic Acid | 115 | 16.354 |
| Irgacure | 0.51635 | 0.07343 |
| Tegbm | 2.3186 | 0.32972 |
| Methyl ethyl hydroquinone | 0.12 | 0.01706 |
| Total | 703.20 | 100.04 |

A removable release liner 16 covers the portion of biomedical electrode 10 containing electrically conductive adhesive 14 to protect the electrically conductive adhesive 14 until it is desired to use the biomedical electrode 10. In a preferred embodiment, release liner 16 is a Polyslik™ material as manufactured by James River Corporation, H. P. Smith Division, Bedford Park, Illinois.

A separately shipped electrical lead wire 18 (not shown in Figure I for clarity) may be either secured to electrically conductive adhesive 14 and then applied to the body or positioned on the body and covered with the biomedical electrode 10 by electrically conductive adhesive 14 after release liner 16 has been removed. Lead wire 18 has an electrically conductive portion 20 and an electrically insulative portion 22. As lead wire 18 is applied to electrically conductive adhesive 14, it is necessary that conductive portion 20 contact electrically conductive adhesive 14. It is preferred that conductive portion 20 of lead wire 18 not extend beyond the edge of protective web 12 in order that only insulative portion 22 of lead wire 18 extend beyond the biomedical electrode 10. Lead wire 18 may be a copper wire whose insulative portion 22 is insulated with any suitable insulation, as for example, rubber or plastic. The end of conductive portion 20 of lead wire 18 is a flat crimped on conductor plate. The conductor plate is flat which facilitates the biomedical electrode 10 being of low profile, flat and conformable to the body. The conductor plate may, for example, be a zinc plated copper or, optionally, a silver plated copper with a chloride treatment.

Figure 3 illustrates an expanded side view of another embodiment of the biomedical electrode 10 of the present invention. In this embodiment, biomedical electrode 10 has a protective web 12, and electrically conductive adhesive 14, a release liner 16 and an electrical lead wire 18 as in the embodiments illustrated in Figures 1 and 2. The embodiment illustrated in Figure 3 also has a conductive film 24 positioned between the protective web 12 and electrically conductive adhesive 14 held in place with a pressure sensitive adhesive 28. The purpose of conductive film 24 is to promote distribution of the current flowing to or from lead wire 18 over the entire surface area of biomedical electrode 10.

Conductive film 24 covers a large portion of the surface area of biomedical electrode 10 and, in a preferred embodiment, covers the entire surface of biomedical electrode 10. Conductive film 24 is secured to protective web 12 with a pressure sensitive adhesive 28 which may be an acrylate adhesive. The purpose of conductive film 24 is to disburse the current delivered by or received by biomedical electrode 10 over a larger portion of surface area of biomedical electrode 10. In order to do this, it is preferred that conductive film 24 be at least as conductive and preferably more conductive than conductive adhesive 14 which is used to secure and provide electrical contact with a body to which biomedical electrode 10 may be attached. In a preferred embodiment, the conductive film 24 is an ethylene vinyl acetate loaded with approximately 28% carbon or a Velostat ® film manufactured by Minnesota Mining and Manufacturing Company, Saint Paul, Minnesota in either case coated top side with a $7 \times 10^{-8}$ to $1.2 \times 10^{-7}$ meters thick vapor coat layer of aluminum or silver. In a preferred embodiment, conductive film 24 is approximately 3 mils (0.76 millimeters) thick. As indicated, conductive adhesive 14 operates to secure biomedical electrode 10 to the body and to provide through electrical conductivity from the body to biomedical electrode 10 or visa versa.

An alternative embodiment of the biomedical electrode 10 of the present invention is illustrated in an expanded side view in Figure 4. This embodiment of biomedical electrode 10 has a protective web 12, an electrically conductive adhesive 14, a release liner 16, an electrical lead wire 18 a conductive film 24 and a pressure sensitive adhesive 28 as in Figure 3. In the embodiment illustrated in Figure 3, the conductive portion 20 of lead wire 18 directly contacts the body since upon the use release liner 16 is removed and discarded. In certain uses of the biomedical electrode, as for example, TENS uses, it may be desirable not to have the conductive portion 20 of lead wire 18 directly contact the body. If the conductive portion 20 of lead wire 18 were electrically insulated from the body, then the current being supplied by lead wire 18 would have to pass through electrically conductive adhesive 14 and be disbursed over the surface area of the electrode by conductive film 24 and then to be supplied to the body again through electrically conductive adhesive 14. In Figure 4, insulator 26, being large enough to cover conductive portion 20 of lead wire 18 but smaller than the surface area of electrically conductive adhesive 14, is positioned between lead wire 18 and release liner 16. In one embodiment, insulator 26 may be larger than the conductive portion 20 of lead wire 18, but still smaller than the surface area of electrically conductive adhesive 14, and, thus, be secured to the biomedical electrode 10 by electrically conductive adhesive 14. In another embodiment, insulator 26 may be secured directly to conductive portion 20 at lead wire 18 as with a pressure sensitive adhesive (not shown). Insulator 26 may be constructed of any of a variety of electrically insulative relatively flat insulators such as a vinyl coating or enamel paint.

Figure 5 is an expanded side view of another embodiment of biomedical electrode 10. As in the embodiment illustrated in Figure 4, biomedical electrode 10 has a protective web 12, an electrically conductive adhesive 14, a release liner 16, an electrical lead wire 18 having a conductive portion 20 and an insulative portion 22 and an insulator 26. This embodiment is similar to the embodiment illustrated in Figure 2 in that optional electrically conductive film 24 is not utilized. Further, in the embodiment illustrated in Figure 5, a pressure sensitive adhesive 28 is utilized between protective web 12 and electrically conductive adhesive 14 in order to further secure the biomedical electrode together by promoting the adherence between protective web 12 and electrically conductive adhesive 14. In a preferred embodiment, the pressure sensitive adhesive 28 is an acrylate adhesive.

Thus, it can be seen that there has been shown and described a novel, flat, biomedical electrode. It is to be understood, however, that various changes, modifications and substitutions in the form and the details of the described invention can be made by those skilled in the art without departing from the scope of the invention as defined by the following claims.

## Claims

I. A biomedical electrode adapted to be applied to a body, comprising:

a protective web having a top side adapted to be oriented away from said body and a bottom side adapted to be oriented toward said body;

an electrically conductive adhesive positioned adjacent said bottom side of said protective web, covering at least a portion of said protective web;

a lead wire having one end with an exposed electrically conductive portion positioned adjacent to said electrically conductive adhesive opposite said protective web;

a removeable liner positioned adjacent said lead wire, being secured by and covering said electrically conductive adhesive;

whereby said removable liner may be removed and said biomedical electrical electrode may be applied to said body.

2. A biomedical electrode as in claim I which further comprises an electrically conductive layer positioned between said protective web and said electrically conductive adhesive and a pressure sensitive adhesive securing said protective web and said electrically conductive layer together.

3. A biomedical electrode as in claim 2 wherein said protective web is electrically insulative.

4. A biomedical electrode adapted to be applied to a body, comprising:

a protective web having a top side adapted to be oriented away from said body and a bottom side adapted to be oriented toward said body;

an electrically conductive adhesive positioned adjacent said bottom side of said protective web, covering at least a portion of said protective web;

a lead wire having one end with an exposed electrically conductive portion positioned adjacent to said electrically conductive adhesive opposite said protective web;

an electrically insulating layer positioned adjacent said one end of said lead wire opposite said electrically conductive adhesive and covering said exposed electrically conductive portion of said one end of said lead wire; and

a removeable liner positioned adjacent said electrically insulating layer, being secured by and covering said electrically conductive adhesive;

whereby said removeable liner may be removed and said biomedical electrical electrode may be applied to said body.

5. A biomedical electrode as in claim 4 which further comprises an electrically conductive layer positioned between said protective web and said electrically conductive adhesive and a layer of pressure sensitive adhesive securing said protective web and said electrically conductive layer together.

6. A biomedical electrode as in claim 5 wherein said protective web is electrically insulative.

7. A biomedical electrode adapted to be applied to a body, comprising:

a protective web having a top side adapted to be oriented away from said body and a bottom side adapted to be oriented toward said body;

a layer of pressure sensitive adhesive positioned adjacent said bottom side of said protective web, covering said protective web;

an electrically conductive adhesive positioned adjacent said layer of pressure sensitive adhesive opposite from said protective web, covering an area of at least a portion of said protective web;

a lead wire having one end with an exposed electrically conductive portion positioned adjacent to said electrically conductive adhesive opposite said protective web;

an electrically insulating layer positioned adjacent said one end of said lead wire opposite said electrically conductive adhesive and covering said exposed electrically conductive portion of said one end of said lead wire; and

a removeable liner positioned adjacent said electrically insulating layer, being secured by and covering said electrically conductive adhesive;

whereby said removeable liner may be removed and said biomedical electrical electrode may be applied to said body.

8. A biomedical electrode as in claim 7 wherein said protective web is electrically insulative.

FIG.1

FIG.2

FIG.4

FIG. 5

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 097 436 (MINNESOTA MINING AND MANUFACTURING CO.) * page 3, line 2 - page 6, line 17; figures 1, 2 * | 1-6 | A 61 B 5/04 A 61 N 1/04 |
| A | | 7,8 | |
| A | WO-A-8 102 097 (MINNESOTA MINING AND MANUFACTURING CO.) * page 8, line 1 - page 9, line 7; figures 1, 2 * | 1-8 | |
| A | EP-A-0 085 327 (MEDTRONIC INC.) * page 12, line 2 - page 13, line 13, line 27; figures 1-3 * | 1,3,4,6 -8 | |

**TECHNICAL FIELDS SEARCHED (Int. Cl.4)**

A 61 B 5/00
A 61 N 1/00

The present search report has been drawn up for all claims

| Place of search | Date of completion of the search | Examiner |
|---|---|---|
| BERLIN | 03-11-1987 | WEIHS J.A. |

EPO FORM 1503 03.82 (P0401)